# EUROPÄISCHE PATENTSCHRIFT

(11) **EP 1 458 621 B1**
(45) Veröffentlichungstag und Bekanntmachung des Hinweises auf die Patenterteilung: **07.01.2009**
(21) Anmeldenummer: 03702401.5
(22) Anmeldetag: 08.01.2003
(51) Int. Cl.: B65D 47/18

(54) **VENTIL ZUR TROPFENAPPLIKATION**
VALVE FOR THE APPLICATION OF DROPS
VALVE POUR L'APPLICATION D'UN LIQUIDE GOUTTE PAR GOUTTE

(30) Priorität: 09.01.2002 DE 10200519
(43) Veröffentlichungstag der Anmeldung: 22.09.2004
(73) Patentinhaber: GAPLAST GmbH, D-82442 Altenau (DE); Backes, Claus H., 66113 Saarbrücken (DE)
(72) Erfinder: BACKES, Claus-H., 66113 Saarbrücken (DE)
(74) Vertreter: Manitz, Finsterwald & Partner GbR
(86) Internationale Anmeldenummer: PCT/EP2003/000111
(87) Internationale Veröffentlichungsnummer: WO 2003/057587

(56) Entgegenhaltungen:
- EP-A- 0 602 019
- WO-A-01/00498
- WO-A-99/55594
- PATENT ABSTRACTS OF JAPAN vol. 1999, no. 14, 22. Dezember 1999 (1999-12-22) -& JP 11 240553 A (YOSHINO KOGYOSHO CO LTD), 7. September 1999 (1999-09-07)

## Beschreibung

Die Erfindung betrifft ein Ventil zur Applikation von flüssigen bzw. in Flüssigkeiten gelösten Arzneistoffen, insbesondere von Augentropfen, nach dem Oberbegriff desAnspruchs 1 (WO 99 55594A).

Tropfersysteme zur Tropfenapplikation sind als Bestandteil von Arzneimittelverpackungen in vielfältigen Ausführungsformen bekannt. Nachteilig an den bislang bekannten Tropfersystemen ist, dass nach einer erstmaligen Ingebrauchnahme eine Verkeimung der zu applizierenden Flüssigkeit nicht wirksam verhindert wird, da bei abgenommener Verschlusskappe Keime durch den Austragskanal in den Arzneistoffvorrat gelangen können. Aus diesem Grund werden bislang dem Arzneistoff Konservierungsmittel beigefügt, welche die Kontaminierung des Arzneistoffes während eines begrenzten Entnahmezeitraumes verhindern soll. Derartige Konservierungsmittel können jedoch beim Anwender allergische Reaktionen hervorrufen, so dass es wünschenswert ist, auf derartige Zusatzstoffe zu verzichten.

Die WO 01/00498 A, die wie die WO 9955594 A dem Oberbegriff des Anspruchs 1 entspricht, offenbart ein Ventil für einen komprimierbaren Behälter mit einem Aufnahmekörper, der einen Austragskanal für Flüssigkeit aufweist, wobei in dem Aufnahmekörper eine einzige elastisch verformbare Membran angeordnet ist, die an ihrem vorderen Ende geschlossen ist und die den Austragskanal abdichtend verschließt.

Aus der JP 11240553 A ist ein Ventil mit einem Aufnahmekörper bekannt, der einen Austragskanal für Flüssigkeit aufweist, wobei in dem Aufnahmekörper eine einzige elastisch verformbare Membran angeordnet ist, die den Austragskanal abdichtend verschließt und die an ihrem vorderen Ende eine Belüftungsöffnung aufweist. In einer Stützplatte für die Membran ist ein weiterer Entlüftungskanal vorgesehen.

Es ist die Aufgabe der vorliegenden Erfindung, ein einfaches Ventil zur Applikation eines Arzneistoffes zu schaffen, das auch nach erstmaliger Ingebrauchnahme eine Verkeimung des flüssigen Arzneimittels wirksam verhindert.

Die Lösung dieser Aufgabe erfolgt durch die Merkmale des Anspruchs 1.

Bei dem erfindungsgemäßen Ventil ist die Möglichkeit geschaffen, die zu applizierende Flüssigkeit durch einen abgedichteten Kanal auszutragen.

Gleichzeitig besteht das erfindungsgemäße Ventil aus nur zwei Funktionsbauteilen, nämlich dem Aufnahmekörper und der Membran.

Um den Zutritt von Mikroorganismen in den Arzneimittelvorrat zu verhin-dern, kann der Austragskanal und auch der Belüftungskanal mit Hilfe der elastisch verformbaren Membran keimdicht verschlossen werden. Nur im Moment der Produktentnahme wird der Austragskanal durch die Membran geöffnet und im Anschluss an die Produktentnahme wird der Austragskanal wieder keimdicht verschlossen.

Da zur Vermeidung eines Unterdruckes im Behälter die bei der Entnahme ausgetriebene Produktmenge durch Umgebungsluft substituiert werden soll, kann diese durch den ebenfalls von der elastischen Membran verschlossenen Belüftungskanal nachgeführt werden. Besonders wirksam wird hierbei eine Kontamination des Behälterinhalts verhindert, wenn vor der Mündung des Belüftungskanales im Aufnahmekörper ein Sterilfilter vorgesehen ist, so dass die Umgebungsluft zunächst den Sterilfilter passieren muss, bevor diese in den Behälter nachströmen kann. Vorteilhafte Ausführungsformen der Erfindung sind in der Beschreibung, den Zeichnungen sowie den Unteransprüchen beschrieben.

Nach einer vorteilhaften Ausführungsform steht die Membran an ihrem vorderen Ende aus dem Aufnahmekörper vor, so dass das vordere Ende der Membran gleichzeitig als elastische und damit weiche Applikationsspitze verwendet werden kann.

Auch ist es vorteilhaft, wenn die Membran an ihrem vorderen Ende domförmig gewölbt ist. Auf diese Weise ist eine Tropfenablösung begünstigt und Verletzungen im Bereich der Applikationsfläche, insbesondere Augenverletzungen, sind ausgeschlossen.

Nach einer weiteren vorteilhaften Ausführungsform liegen Membran und Aufnahmekörper entlang eines Anlageabschnittes abdichtend aneinander an, wobei der Anlageabschnitt insbesondere als Rotationsparaboloid ausgebildet sein kann. Eine solche Formgebung hat sich im Hinblick auf ein sicheres Öffnen und Verschließen im Bereich des Anlageabschnittes als vorteilhaft herausgestellt.

Bevorzugt ist die Membran als einseitig offener Hohlkörper ausgebildet, so dass bei geringem Materialaufwand eine gute Elastizität und damit gute Schließ- und Dichteigenschaften gewährleistet sind.

Nach einer weiteren vorteilhaften Ausführungsform ist die Membran rotationssymmetrisch ausgebildet und im Inneren der Membran ist ein Stützkörper angeordnet, der die Membran in axialer Richtung vorspannt. Ein derartiger Stützkörper kann dazu beitragen, dass die Membran gegen den Anlageabschnitt des Aufnahmekörpers dichtend angedrückt wird, wobei dennoch bei Aufbringen des Austragsdruckes ein Ablösen der Membran von dem Aufnahmekörper entgegen der Vorspannkraft erfolgt.

Eine besonders einfach und damit kostengünstig herzustellende Ausführungsform ist gegeben, wenn das Ventil aus nur drei Funktionsbauteilen besteht, nämlich dem Aufnahmekörper, der Membran und dem Stützkörper. Die einzigen Bauteile, die abgesehen von dem Behälter noch benötigt werden, sind eine Abdeckkappe und ein optionaler Sterilfilter. Diese Bauteile sind jedoch keine funktionsnotwendigen Bauteile des Ventils.

Bei einer weiteren vorteilhaften Ausführungsform der Erfindung sind in dem Ventil mehrere Ringkanäle vorgesehen, die über Überströmkanäle miteinander in Verbindung stehen. Beispielsweise kann zwischen Stützkörper und Membran ein erster Ringkanal und zwischen Aufnahmekörper und Membran ein zweiter Ringkanal vorgesehen sein, die miteinander über einen Überströmkanal in der Membran in Verbindung stehen. Bei dieser Ausführungsform wird die auszutragende Flüssigkeit zunächst in einen Ringraum zwischen Stützkörper und Membran und anschließend in einen Ringraum zwischen Membran und Aufnahmekörper gefördert. Bei Erhöhen des Austragsdruckes kann dann die Flüssigkeit von dem zweiten Ringkanal durch den Anlageabschnitt hindurch in Richtung eines Auslaufkanales am vorderen Ende der Membran bzw. des Aufnahmekörpers gefördert werden.

Eine besonders einfache Montage des Ventils ist gegeben, wenn der Stützkörper im Inneren der Membran über ein Rastmittel mit der Membran in Eingriff steht, beispielsweise eine umlaufende Rastlippe. Auf diese Weise muss lediglich die Membran über den Stützkörper gezogen und verrastet werden, wodurch diese beiden Bauteile bereits zusammengefügt sind. Eine einfache Montage dieser so vormontierten Baugruppe kann dadurch erfolgen, dass der Stützkörper mit aufgesetzter Membran in den Aufnahmekörper eingesetzt und über ein weiteres Rastmittel in dem Aufnahmekörper befestigt wird. Hierdurch ist ein voll funktionsfähiges Ventil mit nur zwei Montageschritten geschaffen.

Bevorzugt erstreckt sich der Stützkörper an seinem hinteren Ende über den gesamten Innenquerschnitt des Aufnahmekörpers, wobei in diesem Bereich eine Durchflussöffnung für die auszutragende Flüssigkeit vorgesehen sein kann. Auf diese Weise ist eine sichere Halterung des Stützkörpers und der Membran gewährleistet.

Das erfindungsgemäße Ventil eignet sich auch für einen Behälter, der zumindest bereichsweise elastisch verformbar ist, beispielsweise für eine Quetschflasche. Grundsätzlich besteht jedoch auch die Möglichkeit, das Ventil für starre Behälter einzusetzen. In diesem Fall muss der Austreibdruck anderweitig erzeugt werden.

Nachfolgend wird die vorliegende Erfindung rein beispielhaft anhand einer vorteilhaften Ausführungsform und unter Bezugnahme auf die beigefügten Zeichnungen beschrieben. Es zeigen:
- Fig. 1: eine Querschnittsansicht eines in einem Behälter befestigten Ventils;
- Fig. 2: eine vergrößerte Darstellung des Ventils von Fig. 1 während eines Produktaustrags; und
- Fig. 3: das Ventil von Fig. 2 während des Druckausgleichs.

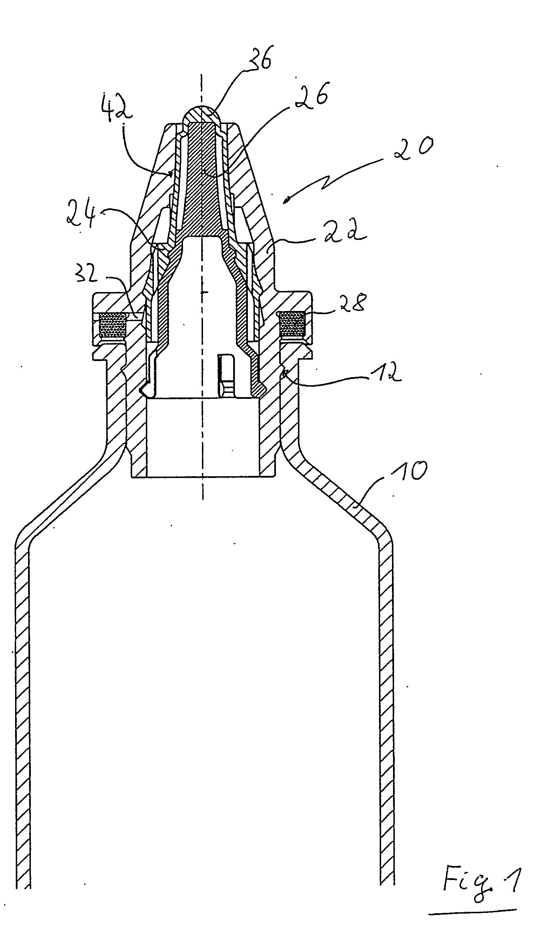

Fig. 1 zeigt im Querschnitt einen Behälter 10 in Form einer Quetschflasche, in dessen Flaschenhals ein Ventil 20 über eine Pressverbindung 12 abgedichtet eingesetzt ist. Das Ventil 20 besteht aus drei Funktionsbauteilen, nämlich einem Aufnahmekörper 22, einer elastisch verformbaren Membran 24 und einem Stützkörper 26.

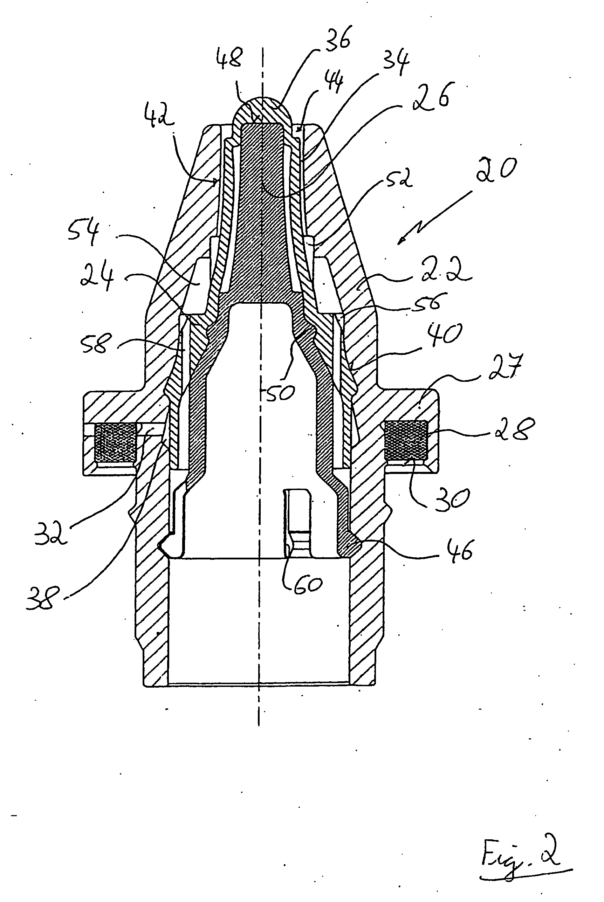

Fig. 2 zeigt eine vergrößerte Querschnittsansicht des Ventils 20. Wie zu erkennen ist, ist der Aufnahmekörper 22 rotationssymmetrisch ausgebildet und besteht aus einem einstückigen Kunststoffbauteil, das sich im oberen Drittel konisch verjüngt. Etwa in der Mitte des Aufnahmekörpers 22 ist ein ringförmiger Vorsprung 27 angeformt, wobei in dem Vorsprung 27 eine ringförmige Nut vorgesehen ist, in die ein Sterilfilter 28 eingesetzt ist. Der Sterilfilter steht einerseits mit seiner unteren Stirnseite 30 mit der Umgebungsluft in Verbindung. Andererseits kann die den Sterilfilter 28 durchströmende Luft über einen Belüftungskanal 32 in das Innere des Aufnahmekörpers 22 gelangen. Am inneren Umfang des Aufnahmekörpers 22 ist ferner im Bereich des Belüftungskanals 32 eine ringförmige Einziehung 38 vorgesehen.

Der Aufnahmekörper 22 ist in seinem Inneren hohl ausgebildet und weist beginnend von seinem unteren Ende einen hohlzylindrischen Abschnitt auf, der sich im oberen Drittel entsprechend der Außenkontur des Aufnahmekörpers verjüngt. Ferner ist im Bereich des vorderen Endes des Aufnahmekörpers 22 ein Austragskanal 34 vorgesehen, durch den die zu applizierende Flüssigkeit ausgetragen werden kann.

Die in das Innere des Aufnahmekörpers 22 eingesetzte Membran 24, die aus einem Elastomer hergestellt ist, ist ein einstückiges, rotationssymmetrisches Bauteil, das langgestreckt, innen hohl und an seinem vorderen Ende verschlossen ist. Im Bereich des verschlossenen vorderen Endes ist die Membran 24 domförmig zu einer etwa halbkugelförmigen Kuppel 36 gewölbt, wobei die Membran 24 mit ihrer Kuppel 36 aus dem Aufnahmekörper 22 vorsteht.

An ihrem unteren Ende ist die Membran 24 im Bereich der Einziehung 38 hohlzylindrisch ausgebildet und überdeckt die Einziehung 38 dichtend. Oberhalb der Einziehung 38 ist am Innenumfang des Aufnahmekörpers 22 eine weitere Einziehung 40 vorgesehen, in die ein komplementär ausgebildeter Rastabschnitt der Membran 24 verrastend eingreift.

Die Membran 24 liegt im Bereich des Austragskanales 34 im Normalzustand (vgl. Fig. 1) dichtend im Bereich eines Anlageabschnittes 42 am Innenumfang des Aufnahmekörpers 22 an. Im Bereich dieses Anlageabschnittes 42 ist die innere Mantelfläche des Austragskanales 34 als Rotationsparaboloid gestaltet, wobei die Membran 24 in diesem Abschnitt an ihrem Außenumfang die gleiche rotationsparabolische Außenkontur aufweist. Am oberen Ende des Austragskanales 34 ist im Bereich der Kuppel 36 ein diskontinuierlicher Übergang zwischen der rotationsparabolischen Außenkontur der Membran 24 und der Kuppel 36 gegeben, wodurch ein Auslaufkanal 44 gebildet wird.

In das Innere der Membran 24 ist der Stützkörper 26 eingesetzt, der ebenfalls ein aus Kunststoff einstückig hergestelltes rotationssymmetrisches Bauteil ist. Der Stützkörper 26 dient einerseits dazu, die Membran 24 im Bereich des Anlageabschnittes 42 axial vorzuspannen und andererseits dazu, die Membran im Aufnahmekörper 22 zu halten. Zu diesem Zweck ist der Stützkörper an seinem unteren Ende mit einem ringförmigen Vorsprung 46 versehen, der in eine entsprechend ausgeführte Ringnut im Inneren des Aufnahmekörpers 22 eingerastet werden kann. An seinem oberen Ende erstreckt sich der Stützkörper bis in den Bodenbereich der Kuppel 36. Dort ist er in einer hohlzylindrischen Vertiefung 48 am inneren vorderen Ende der Membran 24 aufgenommen.

Im Bereich des Anlageabschnittes 42 ist der Stützkörper 26 stiftförmig ausgebildet, wobei die Außenkontur des stiftförmigen Abschnittes ebenfalls als Rotationsparaboloid ausgebildet ist. Wie die Figuren jedoch zeigen, besteht zwischen der äußeren Mantelfläche des stiftförmigen Abschnittes des stützkörpers 26 und der inneren Umfangsfläche der Membran 24 in diesem Abschnitt ein deutlicher Abstand, der es gestattet, dass sich die Membran nach innen in Richtung des Stützkörpers 26 verformt (vgl. Fig. 2).

Etwa in der Mitte ist der Stützkörper 26 an seinem Außenumfang mit einer umlaufenden Rastlippe 50 versehen, die in eine komplementär ausgebildete, ringförmige Rastvertiefung am Innenumfang der Membran 24 verrastend eingreift. Hierdurch ist die Membran zwischen dem Boden der Kuppel 36 und der Rastlippe 50 axial leicht gespannt.

Wie Fig. 1 zeigt, ist die Membran 24 mit dem Stützkörper 26 so in den Aufnahmekörper 22 eingesetzt, dass bei verrastetem Stützkörper 26 und bei verrasteter Membran 24 die Membran entlang des Anlageabschnittes 42 flächig und dichtend am Innenumfang des Aufnahmekörpers 22 anliegt. An der inneren Mündung des Anlageabschnittes 42 ist im Aufnahmekörper 22 ein Ringkanal 52 ausgebildet (Fig. 2), dem mindestens ein axialer Überströmkanal 54 vorausgeht. Diesem geht wiederum ein weiterer Ringkanal 56 voraus. Sowohl der Ringkanal 52 wie auch der Überströmkanal 54 und der Ringkanal 56 sind am Außenumfang von dem Aufnahmekörper 22 und am Innenumfang von der Membran 24 begrenzt.

Wie insbesondere Fig. 2 zeigt, sind in der Membran 24 mehrere axiale Überströmkanäle 58 vorgesehen, die das Innere der Membran 24 mit der Außenseite der Membran verbinden. Hierdurch kann Flüssigkeit vom Inneren des Aufnahmekörpers 22 in den Ringkanal 56 strömen.

Der Stützkörper 26 weist ferner an seiner Unterseite mehrere Durchflussöffnungen 60 auf, die über den Umfang des Stützkörpers 26 verteilt sind und sich in axialer Richtung erstrecken.

Die Funktionsweise des in den Fig. 1 bis 3 dargestellten Ventils ist wie folgt:

Im Ruhezustand befindet sich die Membran 24 des Ventils 20 in der in Fig. 1 dargestellten Position, in der die Membran 24 sowohl den Austragskanal 34 (Fig. 2) wie auch den Belüftungskanal 32 nach außen abgedichtet verschließt, so dass keine Flüssigkeit aus dem Inneren des Behälters 10 austreten und keine kontaminierte Luft in das Innere des Behälters 10 eindringen kann.

Zum Austragen von Flüssigkeit aus dem Behälter 10 wird ein gewünschter Austragsdruck aufgebracht, beispielsweise durch Zusammenquetschen des zumindest bereichsweise elastisch verformbaren Behälters 10. Hierdurch entsteht im Inneren des Behälters 10 ein Überdruck, der die im Behälter 10 befindliche Flüssigkeit durch den Austragskanal 34 austreibt (vgl. Fig. 2). Hierbei strömt die Flüssigkeit zunächst in das Innere des Aufnahmekörpers 22, von dort durch die Durchflussöffnungen 60 in das Innere der Membran 24 und von dort durch die Überströmkanäle 58 in den Ringkanal 56. Von dort gelangt die Flüssigkeit durch den Überströmkanal 54 in den Ringkanal 52, wobei der von der Flüssigkeit auf die Membran 24 ausgeübte Druck die Membran von dem Aufnahmekörper 22 nach Innen in Richtung des Stützkörpers 26 abhebt, so dass zwischen der Membran 24 und dem Aufnahmekörper 22 ein Ringspalt entsteht, wie dies in Fig. 2 dargestellt ist. Anschließend gelangt die Flüssigkeit in den Auslaufkanal 44 und sammelt sich an der Kuppel 36 als Tropfen, so dass ein Applizieren des Tropfens, beispielsweise im Auge eines Anwenders, möglich ist.

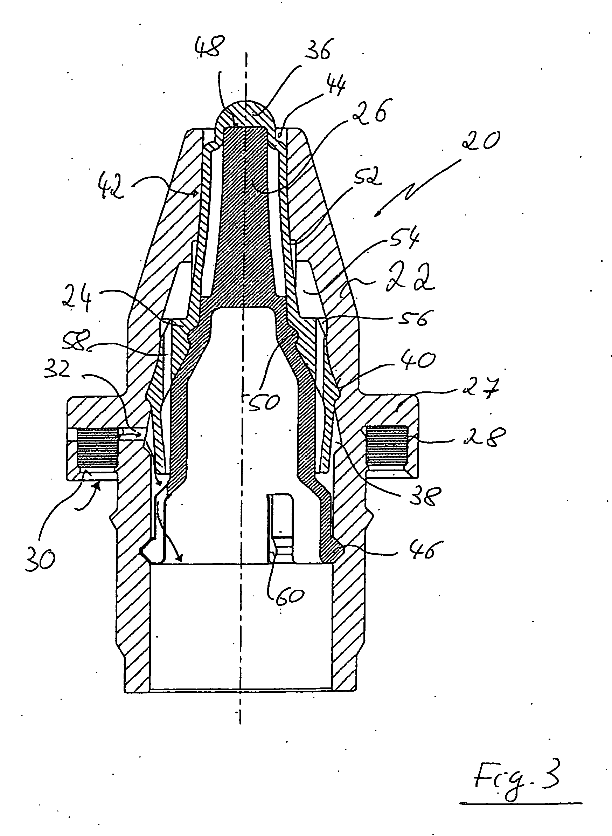

Fig. 3 zeigt den Zustand der Membran 24, nachdem der Austragsdruck zurückgenommen worden ist. Hierdurch ist der Überdruck im Inneren des Behälters 10 abgebaut und die Rückstellkräfte der vorgespannten elastischen Membran 24 bewirken, dass diese wieder fest an der Innenmantelfläche des Anlagebereichs 42 des Aufnahmekörpers 22 anliegt, so dass der Austragskanal 34 wieder abgedichtet verschlossen ist.

Der im Behälter 10 entstandene Unterdruck, der durch das Austragen der Flüssigkeit erzeugt ist, bewirkt anschließend, dass sich der untere Randbereich der Membran 24, der zuvor den Belüftungskanal 32 bzw. die Einziehung 38 abgedichtet verschlossen hat, vom Innenumfang des Aufnahmekörpers 22 abhebt, wie dies in Fig. 3 dargestellt ist. Hierdurch kann Umgebungsluft über den Sterilfilter 28 und den Belüftungskanal 32 und die Einziehung 38 in das Innere des Behälters 10 nachströmen, bis ein Druckausgleich zwischen Umgebungsdruck und Behälterinnendruck erreicht ist. Die entsprechende Luftströmung ist in Fig. 3 mit Pfeilen angedeutet.

Wird das Ventil in einem Tropfersystem eingesetzt, das keinen Druckausgleich erfordert, kann der Belüftungskanal 32 und der Sterilfilter 28 weggelassen werden.

### Bezugszeichenliste

- 10: Behälter
- 12: Pressverbindung
- 20: Ventil
- 22: Aufnahmekörper
- 24: Membran
- 26: stützkörper
- 27: Vorsprung
- 28: Sterilfilter
- 30: untere Stirnseite
- 32: Belüftungskanal
- 34: Austragskanal
- 36: Kuppel
- 38: ringförmige Einziehung
- 40: Einziehung
- 42: Anlageabschnitt
- 44: Auslaufkanal
- 46: Vorsprung
- 48: Vertiefung
- 50: Rastlippe
- 52: Ringkanal
- 54: Überströmkanal
- 56: Ringkanal
- 58: Überströmkanal
- 60: Durchflussöffnung

## Patentansprüche

1. Ventil zur Tropfenapplikation einer in einem Behälter aufbewahrten Flüssigkeit, mit einem Aufnahmekörper (22), der einen Austragekanal für die Flüssigkeit aufweist, wobei in dem Aufnahmekörper (22) eine einzige elastisch verformbare Membran (24) angeordnet ist, die an ihrem vorderen Ende geschlossen ist und den Austragskanal (34) abdichtend verschließt,
**dadurch gekennzeichnet, dass**
der Aufnahmekörper (22) einen Belüftungskanal (32) für nachströmende Luft aufweist, wobei die Membran (24) auch den Belüftungskanal (32) abdichtend verschließt, der sich durch den Aufnakmekörper (22) erstreckt.

2. Ventil nach Anspruch 1,
**dadurch gekennzeichnet, dass**
die Membran (24) domförmig gewölbt ist.

3. Ventil nach Anspruch 1,
**dadurch gekennzeichnet, dass**
die Membran (24) an ihrem vorderen Ende aus dem Aufnahmekörper (22) vorsteht.

4. Ventil nach Anspruch 1,
**dadurch gekennzeichnet, dass**
die Membran (24) als einseitig offener Hohlkörper ausgebildet ist.

5. Ventil nach Anspruch 1,
**dadurch gekennzeichnet, dass**
im Inneren der Membran ein Stützkörper (26) angeordnet ist, der die Membran (24) in axialer Richtung vorspannt.

6. Ventil nach Anspruch 1,
**dadurch gekennzeichnet, dass**
im Inneren der Membran (24) ein Stützkörper (26) vorgesehen ist, der über mindestens ein Rastmittel (50) mit der Membran (24) in Eingriff steht.

7. Ventil nach Anspruch 1,
**dadurch gekennzeichnet, dass**
ein Stützkörper (26) für die Membran (24) vorgesehen ist, der über mindestens ein Rastmittel (46) mit dem Aufnahmekörper (22) in Eingriff steht.

8. Ventil nach Anspruch 1,
**dadurch gekennzeichnet, dass**
ein Stützkörper (26) für die Membran (24) vorgesehen ist, der sich von unterhalb eines in dem Aufnahmekörper (22) vorgesehenen Belüftungskanales (32) bis in den Bereich des oberen Endes (48) der Membran (24) erstreckt.

9. Ventil nach Anspruch 1,
**dadurch gekennzeichnet, dass**
ein Stützkörper (26) für die Membran (24) vorgesehen ist, der sich an seinem unteren Ende über den gesamten Innenquerschnitt des Aufnahmekörpers (22) erstreckt und der in diesem Bereich mindestens eine Durchflussöffnung (60) aufweist.

10. Ventil nach Anspruch 1,
**dadurch gekennzeichnet, dass**
zwischen einem Stützkörper (26) und der Membran (24) ein Ringkanal vorgesehen ist.

11. Ventil nach Anspruch 1,
**dadurch gekennzeichnet, dass**
zwischen Aufnahmekörper (22) und Membran (24) ein Ringkanal (52, 56) vorgesehen ist.

12. Ventil nach Anspruch 10 und 11,
**dadurch gekennzeichnet, dass**
in der Membran mindestens ein Überströmkanal (58) vorgesehen ist, der die beiden Ringkanäle miteinander verbindet.

13. Ventil nach Anspruch 1,
**dadurch gekennzeichnet, dass**
die Membran (24) und der Aufnahmekörper (22) entlang eines Anlageabschnittes (42) abdichtend aneinander anliegen, der insbesondere als Rotationsparaboloid ausgebildet ist.

14. Ventil nach Anspruch 13,
**dadurch gekennzeichnet, dass**
am Anfang des Anlageabschnittes (42) ein Ringkanal (52) und am Ende des Anlageabschnittes (42) ein Auslaufkanal (44) vorgesehen sind.

15. Ventil nach Anspruch 1,
**dadurch gekennzeichnet, dass**
in dem Aufnahmekörper (22) vor der Mündung des Belüftungskanales (32) ein Sterilfilter (28) vorgesehen ist.

16. Ventil nach Anspruch 5,
**dadurch gekennzeichnet, dass**
dieses aus nur drei Funktionsbauteilen zusammengesetzt ist, nämlich dem Aufnahmekörper (22), der Membran (24) und dem Stützkörper (26).

17. Tropfersystem mit einem Behälter (10) und einem Ventil (20) nach einem der vorstehenden Ansprüche, das den Behälter (10) abgedichtet verschließ.

18. Tropfersystem nach Anspruch 17,
**dadurch gekennzeichnet, dass**
der Behälter (10) zumindest bereichsweise elastisch verformbar ist.

## Claims

1. A valve for the drop application of a liquid stored in a container comprising a take-up body (22) which has a discharge passage for the liquid, with a single elastically deformable membrane (24) being arranged in the take-up body (22) which is closed at its front end and sealingly closes the discharge passage (34),
**characterized in that**
the take-up body (22) has an aeration passage (32) for a follow-up air flow, with the membrane (24) also sealingly closing the aeration passage (32) which extends through the take-up body (22).

2. A valve in accordance with claim 1, **characterized in that** the membrane (24) is arched in dome-shape.

3. A valve in accordance with claim 1, **characterized in that** the front end of the membrane (24) projects out of the take-up body (22).

4. A valve in accordance with claim 1, **characterized in that** the membrane (24) is made as a hollow body open at one end.

5. A valve in accordance with claim 1, **characterized in that** a support element (26), which pre-stresses the membrane (24) in the axial direction, is arranged in the interior of the membrane.

6. A valve in accordance with claim 1, **characterized in that** a support element (26), which is in engagement with the membrane (24) via at least one latching means (50), is provided in the interior of the membrane (24).

7. A valve in accordance with claim 1, **characterized in that** a support element (26), which is in engagement with the take-up body (22) via at least one latching means (46), is provided for the membrane (24).

8. A valve in accordance with claim 1, **characterized in that** a support body (26) is provided for the membrane (24) and extends from beneath an aeration passage (32) provided in the take-up body (22) up to and into the region of the upper end (48) of the membrane (24).

9. A valve in accordance with claim 1, **characterized in that** a support body (26) for the membrane (24) is provided whose lower end extends over the total inner cross-section of the take-up body (22) and which has at least one throughflow opening (60) in this region.

10. A valve in accordance with claim 1, **characterized in that** a ring passage is provided between a support body (26) and the membrane (24).

11. A valve in accordance with claim 1, **characterized in that** a ring passage (52, 56) is provided between the take-up body (22) and the membrane (24).

12. A valve in accordance with claim 10 and claim 11, **characterized in that** at least one overflow passage (58) is provided in the membrane and connects the two ring passages to one another.

13. A valve in accordance with claim 1, **characterized in that** the membrane (24) and the take-up body (22) sealingly contact one another along a contact section (42) which is in particular made as a paraboloid of revolution.

14. A valve in accordance with claim 13, **characterized in that** a ring passage (52) is provided at the start of the contact section (42) and an outflow passage (44) is provided at the end of the contact section (42).

15. A valve in accordance with claim 1, **characterized in that** a sterile filter (28) is provided in the take-up body (22) in front of the orifice of the aeration passage (32).

16. A valve in accordance with claim 5, **characterized in that** it is only made up of three functional components, namely of the take-up body (22), of the membrane (24) and of the support body (26).

17. A dropper system comprising a container (10) and a valve (20) in accordance with any one of the preceding claims, which sealingly closes the container (10).

18. A dropper system in accordance with claim 17, **characterized in that** the container (10) is elastically deformable at least regionally.

## Revendications

1. Valve pour l'application au goutte-à-goutte d'un liquide contenu dans un réservoir, comprenant un corps récepteur (22) qui comporte un canal de sortie pour le liquide, dans laquelle une unique membrane élastiquement déformable (24) est agencée dans le corps récepteur (22), laquelle est fermée à son extrémité antérieure et referme de façon étanche le canal de sortie (34),
**caractérisée en ce que** le corps récepteur (22) comporte un canal d'aération (32) pour l'air qui s'écoule à la suite, ladite membrane (24) refermant également de façon étanche le canal d'aération (32), lequel s'étend à travers le corps récepteur (22).

2. Valve selon la revendication 1,
**caractérisée en ce que** la membrane (24) est bombée en forme de coupole.

3. Valve selon la revendication 1,
**caractérisée en ce que** la membrane (24) dépasse à son extrémité antérieure hors du corps récepteur (22).

4. Valve selon la revendication 1,
**caractérisée en ce que** la membrane (24) et réalisée sous la forme d'un corps creux ouvert d'un côté.

5. Valve selon la revendication 1,
**caractérisée en ce qu'**à l'intérieur de la membrane est agencé un corps de soutien (26) qui précontraint la membrane (24) en direction axiale.

6. Valve selon la revendication 1,
**caractérisée en ce qu'**à l'intérieur de la membrane (24) est prévu un corps de soutien (26) qui est en engagement avec la membrane (24) via au moins un organe d'enclenchement (50).

7. Valve selon la revendication 1,
**caractérisée en ce qu'**il est prévu un corps de soutien (26) pour la membrane, qui est en engagement avec le corps récepteur (22) via au moins un organe d'enclenchement (46).

8. Valve selon la revendication 1,
**caractérisée en ce qu'**il est prévu un corps de soutien (26) pour la membrane (24), lequel s'étend depuis le dessous d'un canal d'aération (32) prévue dans le corps récepteur (22) jusque dans la région de l'extrémité supérieure (48) de la membrane (24).

9. Valve selon la revendication 1,
**caractérisée en ce qu'**il est prévu un corps de soutien (26) pour la membrane (24), lequel s'étend à son extrémité inférieure sur la totalité de la section intérieure du corps récepteur (22) et comprend dans cette région au moins une ouverture de passage (60).

10. Valve selon la revendication 1,
**caractérisée en ce qu'**il est prévu un canal annulaire entre un corps de soutien (26) et la membrane (24).

11. Valve selon la revendication 1,
**caractérisée en ce qu'**il est prévu un canal annulaire (52, 56) entre le corps récepteur (22) et la membrane (24).

12. Valve selon la revendication 10 et 11,
**caractérisée en ce qu'**il est prévu dans la membrane au moins un canal de déversement (58) qui relie les deux canaux annulaires l'un à l'autre.

13. Valve selon la revendication 1,
**caractérisée en ce que** la membrane (24) et le corps récepteur (22) sont appliqués l'un contre l'autre de manière étanche le long d'un tronçon d'appui (42), lequel est réalisé en particulier sous forme de paraboloïde de révolution.

14. Valve selon la revendication 13,
**caractérisée en ce qu'**au commencement du tronçon d'appui (42) est prévu un canal annulaire (52) et à la fin du tronçon d'appui (42) est prévu un canal de sortie (44).

15. Valve selon la revendication 1,
**caractérisée en ce qu'**il est prévu un filtre stérile (28) dans le corps récepteur (22) devant l'embouchure du canal d'aération (32).

16. Valve selon la revendication 5,
**caractérisée en ce que** celle-ci est composée de seulement trois composants fonctionnels, à savoir le corps récepteur (22), la membrane (24) et le corps de soutien (26).

17. Système goutte-à-goutte comprenant un réservoir (10) et une valve (20) selon l'une des revendications précédentes, qui referme le réservoir (10) de façon étanche.

18. Système goutte-à-goutte selon la revendication 17,
**caractérisé en ce que** le réservoir (10) est élastiquement déformable au moins par zones.
